# EUROPEAN PATENT APPLICATION

(11) **EP 2 581 095 A1**
(43) Date of publication of application: **17.04.2013**
(21) Application number: 11008321.9
(22) Date of filing: 14.10.2011
(51) Int. Cl.: A61L 2/03, A61L 2/18, B08B 3/10, A61L 2/22, C25B 1/13

(54) **Cleaning surfaces with ozone and electrolysis water**

(71) Applicant: Naumann, Michael, 29320 Hermannsburg (DE)
(72) Inventor: Naumann, Michael, 29320 Hermannsburg (DE)
(74) Representative: Hauck Patent- und Rechtsanwälte

(57) **Abstract**

Apparatus for cleaning a surface comprising
● a source of a catholyte electrochemically activated liquid,
● a source of ozonated water including a water reservoir and an ozone generator,
● a liquid dispenser,
● a first flow path connecting the source of the catholyte electrochemically activated liquid with the liquid dispenser, and
● a second flow path connecting the source of the ozonated water with the liquid dispenser, wherein

the liquid dispenser is adapted to dispense the catholyte electrochemically activated liquid and the ozonated water.

## Description

The invention relates to an apparatus and method for cleaning a surface. In this application, the word "cleaning" is used in a broad sense, including removal of dust or dirt particles from a surface as well as reducing the number of microorganisms or other biologically active species, commonly referred to as "sanitising" and/or "disinfecting". Such cleaning apparatuses and methods are needed in various contexts, for example cleaning of floors in buildings, desks or other furniture, medical applications, food production or handling facilities, pest control systems, and restaurants, to name a few. Many known cleaning methods include application of a liquid cleaning medium to a surface, for example by spraying the cleaning medium onto the surface or by manual application with a brush or cloth.

Many known cleaning media include water and a detergent. The detergent typically includes a solvent, a builder and a surfactant.

From the document US 2007/0186368 A1 a cleaning apparatus having a functional generator for producing electrochemically activated cleaning liquid has become known. The functional generator converts a base liquid into an anolyte electrochemically activated liquid by means of an electrolyser. The catholyte and anolyte electrochemical liquids are combined and dispensed onto a surface to be cleaned.

From document WO 2009/155543 A1 a similar cleaning apparatus has become known, wherein the electrolyser comprises an electrolysis cell having conductive polymer electrodes.

While it is true that the application of electrochemically activated anolyte and catholyte liquids helps to reduce the amount of chemical detergents, there is still room for improvement regarding the cleaning and/or sanitising properties of this type of cleaning liquids.

Based on these observations, it is an object of the invention to provide an apparatus and a method for cleaning a surface that is environmentally friendly and offers improved cleaning and/or sanitising effectiveness.

This problem is solved by the apparatus for cleaning a surface with the features of claim 1 and by the method for cleaning a surface with the steps of claim 11. Preferred aspects of the invention are given in the dependent claims.

The apparatus for cleaning a surface comprises:
● a source of catholyte electrochemically activated liquid,
● a source of ozonated water including a water reservoir and an ozone generator,
● a liquid dispenser,
● a first flow path connecting the source of the electrolyte electrochemically activated liquid with the liquid dispenser, and
● a second flow path connecting the source of the ozonated water with the liquid dispenser, wherein
the liquid dispenser is adapted to dispense the catholyte electrochemically activated liquid and the ozonated water.

The apparatus may be stationary or mobile. It may be used for cleaning any type of surface, for example floors, components of buildings and industrial facilities, patient or animal skin, food products, leaves or fruits of plants, etc.

The source of a catholyte electrochemically activated liquid may be a container or reservoir which is filled with the catholyte electrochemically activated liquid.

The term "electrochemically activated liquid" refers to a liquid, for example water, with elevated reactivity containing reactive species and/or meta-stable ions and free radicals formed after exposure to electrochemical energy. For example, a base liquid may be subjected to an electrical current in an electrolysis cell. The base liquid may be regular tap water, pure water with one or more electrolytes, or any other aqueous solution containing a suitable concentration of electrolytes to obtain a suitable electric conductivity. Suitable electrolytes include salts such as chloride salt, nitrate salt, carbonate salt or any other salt, that is soluble in water. Chloride salts include, for example, sodium chloride and calcium chloride, etc.

By way of applying the electrical current to the base liquid, under suitable conditions, the electrochemical activation leads to an anolyte electrochemically activated liquid and a catholyte electrochemically activated liquid. These liquids are named anolyte or catholyte, respectively, because they are typically formed in the region of an anode or in the region of a cathode, in particular in so-called anode and cathode chambers of an electrolysis cell, for example.

The catholyte electrochemically activated liquid is known to have anti-oxidant characteristics. The catholyte electrochemically activated liquid typically is strongly basic having a pH in the range from about 8 to about 12. Typically, it may also have a negative oxidation-reduction potential below minus 100 mV, for example in a range of about minus 500 mV to about minus 1,000 mV. In total, the catholyte electrochemically activated liquid may not have any substantial electrical charge.

These characteristics are known to impart the catholyte electrochemically activated liquid properties of a surfactant, helping to achieve a cleaning effect.

The term "ozonated water" refers to an aqueous liquid including a certain amount of ozone dissolved therein. Due to the ozone content, the ozonated water has powerful oxidising capabilities. It is understood that the ozonated water may include other oxidising agents such as chlorine dioxide, hydrogene peroxide or peracetic acid in varying amounts. The ozonated water may include any other chemical substances as well, wherein preferably the main oxidising effect is based on the ozone. The ozone concentration of the ozonated water may be in a range of from about 0.2 mg/l to about 5 mg/l, for example, more particularly in a range of from about 0.5 mg/l to about 4 mg/l.

As the ozone of the ozonated water is meta-stable only and has relatively short decay time of about 20 to 30 minutes, the source of ozonated water includes a water reservoir filled with the aqueous base liquid, water in particular, and an ozone generator. The ozone generator may be any known type of ozone generator, for example producing ozone out of ambient air by means of the corona discharge method or the dielectric barrier discharge method, or by using ultraviolet light. The ozone produced by the ozone generator may be in gaseous form and then dissolved in water of the water reservoir. By using other known methods of ozone generation, the ozone may also be produced out of the water contained in the water reservoir, so that no additional step of dissolving the ozone in the water is necessarily performed.

The liquid dispenser may be any device suitable for dispensing the catholyte electrochemically activated liquid and the ozonated water onto the surface. It may be operated manually or may include a pump, for example an electrically driven displacement pump.

The first and second flow paths connecting the source of the catholyte electrochemically activated liquid or the source of the ozonated water, respectively, with the liquid dispenser, may be any means suitable for guiding the liquids from the respective sources to the liquid dispenser, for example hoses or conduits. They may include control means for controlling a flow, such as valves, for example.

With the inventive apparatus, the catholyte electrochemically activated liquid and the ozonated water are dispensed onto the surface to be cleaned. This specific combination of liquids was found to have superior cleaning and sanitising properties, in particular as compared to a separate application of both types of liquids. Without wishing to be bound by theory, it is believed that the superior cleaning and/or sanitising effect is based on an optimised distribution of the ozonated water through the surfactant-like properties of the catholyte electrochemically activated water, which more than compensates for the expected loss of sanitising power of the ozonated water effected by the combination with the catholyte electrochemically activated water. As a result, the inventive apparatus allows for an improved cleaning and/or sanitising effectiveness in a single cleaning step, which is an important practical advantage over prior art methods.

According to an aspect of the invention, the dispenser is adapted to dispense the catholyte electrochemically activated liquid and the ozonated water in a predetermined ratio. For example, the ratio of volumes of catholyte electrochemically activated liquid and ozonated water dispensed in a certain time frame may be 50:50, 60:40, 70:30 etc. To this end, the dispenser may be equipped with separate displacement pumps driven mechanically or electrically, for example, wherein the displacement volumes of both pumps are selected according to the desired ratio of the dispensed liquids. In the alternative, an electronic control means may be used to control and/or regulate the volumes of each of the liquids being dispensed. This helps to optimise the cleaning and/or sanitising effect of the apparatus.

According to an aspect, the dispenser comprises an orifice connected to the first flow path and to the second flow path. In this way, the dispenser is adapted to dispense a mixture of the catholyte electrochemically activated liquid and the ozonated water through a single orifice. The connection to the flow paths may be designed such that both liquids are mixed just before they are being dispensed through the orifice. This helps to ensure that each region of the surface to be cleaned is supplied with the desired, evenly distributed mixture of both liquids, whereas at the same time any undesirable neutralising effect due to bringing both liquids in contact with each other is kept to a minimum.

According to an aspect, the dispenser comprises a first orifice connected to the first flow path and a second orifice connected to the second flow path. In this way, the dispenser is adapted to dispense the catholyte electrochemically activated liquid and the ozonated water through separate orifices, so that both liquids do not get in contact with each other prior to being dispensed through the respective first and second orifices of the dispenser. By selecting a suitable arrangement and design of the first and second orifices, it can nevertheless be achieved that both liquids are supplied evenly to the desired region of the surface to be cleaned, whereas both liquids are mixed or combined with each other only after having passed through the first and second orifices, in particular only when they reach the surface to be cleaned. To this end, it is an option to arrange the first and second orifices in a distance from one another which is larger than 30 % of a distance between the orifices and the surface to be cleaned under typical operating conditions. For example, the distance between the first and second orifices may be 10 cm or more.

Further, the first and second orifices may be arranged such that the catholyte electrochemically activated liquid and the ozonated water expelled through the orifices will essentially target the same region of the surface.

According to an aspect, the dispenser includes pressurising means adapted to supply the catholyte electrochemically activated liquid to the first orifice with a first pressure and the ozonated water to the second orifice with a second pressure, wherein the second pressure is preferably smaller than the first pressure. Using such pressurising means allows to control the amount of each of the liquids to be dispensed so that a desired ratio is obtained. Further, choosing appropriate pressures for each of the liquids can account for certain properties of the liquids, in particular for a limited stability under high pressures. In particular, ozonated water having a limited pressure stability can be supplied with a relatively low second pressure.

According to an aspect, the dispenser comprises at least one spray nozzle. The nozzle may include an orifice through which both liquids are dispensed as a mixture, or it may include a first orifice through which the catholyte electrochemically activated liquid is supplied and a second orifice through which the ozonated water is supplied. The spray nozzle allows to evenly distribute both liquids in small droplets towards the surface. If first and second orifices are employed, which may be integrated in a single spray nozzle or may be designed as openings of corresponding first and second spray nozzles, both liquids can be directed towards the surface such that no substantial interaction of both liquids will occur prior to getting in contact with the surface. This further helps to minimise undesirable neutralisation effects. Also, a spray nozzle optimised for an external mixture of both liquids may be used, for example having a first orifice for one of the liquids and a second orifice for the other liquid, wherein the second orifice is placed adjacent to the first orifice, so that both streams of liquids when expelled through the first and second orifices will hit one another and become mixed and/or atomized. In particular, the first orifice may be arranged centrally and the second orifice may surround the first central orifice, preferably concentrically.

According to an aspect, the ozone generator is an electrolytic ozone generator. Electrolytic ozone generators split water molecules into H₂, O₂ and O₃. The hydrogen gas may be removed. Electrolytic ozone generation is advantageous, because relatively high concentrations of ozone can be achieved. Further, the ozone is produced directly in the water, which may be taken from the water reservoir. For this reason, it is not necessary to perform a separate step of dissolving the ozone produced in gaseous form in the water. The electrolytic ozone generator may include one or more diamond electrodes.

According to an aspect, the source of catholyte electrochemically activated liquids includes a base liquid reservoir and an electrolysis cell being in fluid communication with the base liquid reservoir and having a first output for the catholyte electrochemically activated liquid, the first output being connected to the first flow path. In general, it is an option to prepare the catholyte electrochemically activated liquid externally and to then supply the source of catholyte electrochemically activated liquid of the apparatus therewith, in particular by filling a corresponding tank or other reservoir or container of the apparatus. In the alternative, the electrochemical activation may be performed within the apparatus so that the same is supplied with a corresponding base liquid only which is filled in a base liquid reservoir. The base liquid is then converted into the catholyte electrochemically activated liquid by means of an electrolysis cell. The electrolysis cell at the same time may produce an anolyte electrochemically activated liquid. By providing the apparatus with such a source of catholyte electrochemically activated liquid it is possible to control production of this liquid and to produce the same as needed.

Also, as the catholyte electrochemically activated liquid may be of limited stability, it may be an advantage to prepare the same on demand.

According to an aspect, the electrolysis cell comprises a second output for an anolyte electrochemically activated liquid, the second output being connected to the second flow path. In general, it is not necessary to use any anolyte electrochemically activated liquid produced by the electrolysis cell for the inventive apparatus. In particular, the anolyte electrochemically activated liquid may just be disposed of. According to the described aspect, however, the anolyte electrochemically activated liquid can be supplied to the second flow path, allowing a combination of the anolyte electrochemically activated liquid with the ozonated water. In particular, the ozonated water may be injected or otherwise mixed with the anolyte electrochemically activated liquid in a desired ratio. This allows to make use of the anolyte electrochemically activated liquid for distribution of the ozonated water. The cleaning process may also benefit from an additional oxidising effect of the anolyte electrochemically activated liquid. By controlling the electrolysis cell and by using appropriate electrodes therein, it is also possible to prepare an anolyte electrochemically activated liquid having a very limited acidity only, so that adverse effects of very low pH anolyte electrochemically activated liquids can be avoided. For example, the pH of the anolyte electrochemically activated liquid may be in the range of 6 to 8.

According to an aspect, the apparatus is a hand-held cleaner, a mobile high pressure cleaner, a hard floor cleaner, a soft floor cleaner or a pest control sprayer. The handheld cleaner may be in the form of a spray bottle, for example. The mobile high pressure cleaner may be portable or may include a cart or trolley. The hard floor cleaner may include one or more rotating brushes acting on the surface on which the liquids are dispensed. The soft floor cleaner may further include a vacuum-based extractor for removing the liquids dispensed onto a carpet, for example. The pest control sprayer may be a hand-held or portable device including a backpack for example, being designed to easily spray the liquids onto plants, as commonly applied for distributing pesticides.

The above stated problem is also solved by the method for cleaning a surface with the features of claim 11. The method comprises the following steps:
● providing a catholyte electrochemically activated liquid,
● operating an ozone generator and providing ozonated water,
● dispensing the catholyte electrochemically activated liquid and the ozonated water onto the surface.

Regarding the features of this method, reference is made to the above explanations of the inventive apparatus. The apparatus includes corresponding features and the explanations relating thereto apply to the features of the inventive method as well. The same is true for the advantages of the apparatus as described above, which are also related to the inventive method. Further, the inventive method may include additional steps as described above in relation to the inventive apparatus. In particular, for performing each of the steps of the method, corresponding means as described in relation to the apparatus may be used. For example, for dispensing the catholyte electrochemically activated liquid and the ozonated water onto the surface, the liquid dispenser of the inventive apparatus may be used, and so forth.

In short, the inventive method allows to easily clean a surface and to achieve a superior cleaning and/or sanitising effect.

According to an aspect of the method, the catholyte electrochemically activated liquid and the ozonated water are dispensed in a predetermined ratio and/or in a mixture. Reference is made to the above explanations of the corresponding features of the apparatus.

According to an aspect of the method, the catholyte electrochemically activated liquid and the ozonated water are dispensed separately. This allows to reduce neutralising effects to a minimum, as explained above with regard to the liquid dispenser comprising different orifices.

According to an aspect of the method, the catholyte electrochemically activated liquid is dispensed with a first pressure and the ozonated water is dispensed with a second pressure, wherein the second pressure is preferably less than the first pressure. Regarding this aspect, reference is made to the above explanations of the inventive apparatus comprising pressurising means.

According to an aspect of the method, the step of providing the catholyte electrochemically activated liquid includes operating of an electrolysis cell, the electrolysis cell converting a base liquid into the catholyte electrochemically activated liquid and an anolyte electrochemically activated liquid, and wherein the ozonated water is combined with the anolyte electrochemically activated liquid prior to the step of dispensing the ozonated water onto the surface. For this aspect as well, reference is made to the above explanations of the corresponding features of the inventive apparatus.

In the following, the invention is explained in greater detail on the basis of two embodiments shown in figures.
- Fig. 1: shows a first embodiment of an inventive apparatus to be supplied with a catholyte electrochemically activated liquid produced externally.
- Fig. 2: shows a second embodiment of an inventive apparatus including an electrolysis cell for producing the catholyte electrochemically activated liquid within the apparatus.

For both embodiments the same reference numerals are used to denote corresponding elements.

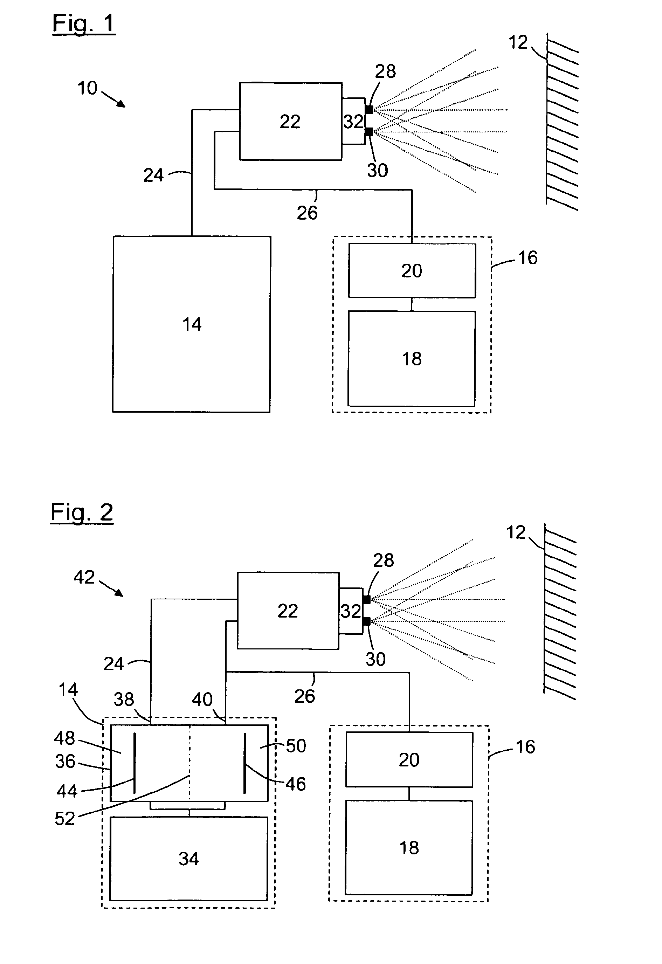

The apparatus 10 for cleaning a surface 12 shown in figure 1 comprises a source 14 of catholyte electrochemically activated liquid in the form of a reservoir.

The apparatus 10 also includes a source 16 of ozonated water having a water reservoir 18 and an ozone generator 20. The water reservoir 18 may be filled with regular tap water, but also with purified water, for example. The ozone generator 20 is an electrolytic ozone generator 20, which is in fluid communication with the water reservoir 18 and uses an electrolytic process to generator ozone directly within the water taken from the water reservoir 18 in a desired amount and/or concentration.

The source 14 of catholyte electrochemically activated water is connected to a liquid dispenser 22 by a first flow path 24 in the form of a conduit. A second flow path 26 connects the liquid dispenser 22 with the source 16 of ozonated water, in particular with an output of the ozone generator 20. The second flow path 26 is a conduit as well.

The liquid dispenser 22 includes a spray nozzle 32, having a first orifice 28 and a second orifice 30. The first orifice 28 is in fluid communication with the first flow path 24. The second orifice 30 is in fluid communication with the second flow path 26. The liquid dispenser 22 also includes pressurising means not shown used to supply the catholyte electrochemically activated water and the ozonated water to the first and second orifices 28, 30 under a desired pressure. The named liquids are supplied separately through these first and second orifices 28, 30, wherein fine droplets of both liquids are distributed evenly over a certain region of the surface 12, such that each portion of the surface 12 is supplied with both types of liquids in a desired ratio.

As stated above, the source 14 of catholyte electrochemically activated water may be any suitable container filled with externally produced catholyte electrochemically activated liquid.

The apparatus 42 for cleaning a surface 12 shown in figure 2 includes a liquid dispenser 22 and a source 16 of ozonated water as described above with regard to the first embodiment of figure 1. It differs in the source 14 of catholyte electrochemically activated liquid which includes a base liquid reservoir 34 and an electrolysis cell 36.

The base liquid reservoir 34 may be filled with regular tap water or pure water, supplemented with a desired composition and amount of electrolytes, as desired to obtain a suitable electrical conductivity.

The electrolysis cell 36 includes a cathode 44 and an anode 46. The cathode 44 is provided within a cathode chamber 48, and the anode 46 is provided within an anode chamber 50. The cathode chamber 48 and the anode chamber 50 are separated from each other by a semi-permeable membrane 52. In particular, the semi-permeable membrane 52 may be permeable for cations, while it may be impermeable for other components of the liquid within the electrolysis cell 36.

The apparatus may also include one or more sources of electrical energy for operating the electrolysis cell 36 and/or for operating any electrical drives of the liquid dispenser 22, and also for operating any electronical control devices not shown. The sources of electrical energy may be batteries, for example.

Both, the cathode chamber 48 and the anode chamber 50, are in fluid communication with the base liquid reservoir 34.

The electrolysis cell 36 includes a first output 38 connected to the first flow path 24, wherein the first output 38 is in fluid communication with the cathode chamber 48, so that catholyte electrochemically activated liquid can be supplied to the first flow path 24. The electrolysis cell 36 comprises a second output 40 connected to the second flow path 26. The second output 40 is in fluid communication with the anode chamber 50, so that an anolyte electrochemically activated liquid can be supplied to the second flow path 26. The second flow path 26 further provides a connection between the liquid dispenser 22 and the source 16 of ozonated water. More precisely, the second flow path 26 is used to inject ozonated water taken from the source 16 of ozonated water into the anolyte electrochemically activated liquid taken from the second output 40 of the electrolyses cell 36, so that a mixture of the anolyte electrochemically activated water and the ozonated water is supplied to the liquid dispenser 22.

## Claims

1. Apparatus (10) for cleaning a surface (12) comprising
● a source (14) of a catholyte electrochemically activated liquid,
● a source (16) of ozonated water including a water reservoir (18) and an ozone generator (20),
● a liquid dispenser (22),
● a first flow path (24) connecting the source (14) of the catholyte electrochemically activated liquid with the liquid dispenser (22), and
● a second flow path (26) connecting the source (14) of the ozonated water with the liquid dispenser (22), wherein
the liquid dispenser (22) is adapted to dispense the catholyte electrochemically activated liquid and the ozonated water.

2. The apparatus according to claim 1, wherein the dispenser (22) is adapted to dispense the catholyte electrochemically activated liquid and the ozonated water in a predetermined ratio.

3. The apparatus according to claim 1 or 2, wherein the dispenser (22) comprises an orifice connected the first flow path (24) and to the second flow path (26).

4. The apparatus according to claim 1 or 2, wherein the dispenser (22) comprises a first orifice (28) connected to the first flow path (24) and a second orifice (30) connected to the second flow path (26).

5. The apparatus according to claim 4, wherein the dispenser (22) includes pressurising means adapted to supply the catholyte electrochemically activated liquid to the first orifice (28) with a first pressure and the ozonated water to the second orifice (30) with a second pressure, wherein the second pressure is preferably smaller than the first pressure.

6. The apparatus according to any of the claims 1 to 5, wherein the dispenser (22) comprises at least one spray nozzle (32).

7. The apparatus according to any of the claims 1 to 6, wherein the ozone generator (20) is an electrolytic ozone generator.

8. The apparatus according to any of the claims 1 to 7, wherein the source (14) of catholyte electrochemically activated liquid includes a base liquid reservoir (34) and an electrolysis cell (36) being in fluid communication with the base liquid reservoir (32) and having a first output (38) for the catholyte electrochemically activated liquid, the first output (38) being connected to the first flow path (24).

9. The apparatus according to claim 8, wherein the electrolysis cell (36) comprises a second output (40) for an anolyte electrochemically activated liquid, the second output (40) being connected to the second flow path (26).

10. The apparatus according to any of the claims 1 to 9, wherein the apparatus is a handheld cleaner, a mobile high pressure cleaner, a hard floor cleaner, a soft floor cleaner, or a pest control sprayer.

11. Method for cleaning a surface (12) comprising the following steps:
● providing a catholyte electrochemically activated liquid,
● operating an ozone generator (20) and providing ozonated water,
● dispensing the catholyte electrochemically activated liquid and the ozonated water onto the surface (12).

12. The method according to claim 11, wherein the catholyte electrochemically activated liquid and the ozonated water are dispensed in a predetermined ratio and/or in a mixture.

13. The method according to claim 11, wherein the catholyte electrochemically activated liquid and the ozonated water are dispensed separately.

14. The method according to claim 13, wherein the catholyte electrochemically activated liquid is dispensed with a first pressure and the ozonated water is dispensed with a second pressure, wherein the second pressure is preferably less than the first pressure.

15. The method according to any of the claims 11 to 14, wherein the step of providing the catholyte electrochemically activated liquid includes operating of an electrolysis cell (36), the electrolysis cell (36) converting a base liquid into the catholyte electrochemically activated liquid and an anolyte electrochemically activated liquid, and wherein the ozonated water is combined with the anolyte electrochemically activated liquid prior to the step of dispensing the ozonated water onto the surface (12).
